# EUROPEAN PATENT SPECIFICATION

(11) **EP 1 363 633 B1**
(45) Date of publication and mention of the grant of the patent: **04.05.2005**
(21) Application number: 02700190.8
(22) Date of filing: 01.03.2002
(51) Int. Cl.: A61K 31/445, A61K 9/20

(54) **A WET GRANULATION PROCESS FOR THE MANUFACTURE OF PHARMACEUTICAL TABLETS CONTAINING PAROXETINE HYDROCHLORIDE ANHYDRATE**
NASSES GRANULATIONSVERFAHREN ZUR HERSTELLUNG VON PHARMAZEUTISCHEN TABLETTEN MIT PAROXETINHYDROCHLORID ANHYDRAT
PROCEDE DE GRANULATION PAR VOIE HUMIDE POUR LA PREPARATION DE COMPRIMES PHARMACEUTIQUES CONTENANT UN HYDROCHLORURE ANHYDRE DE PAROXETINE

(30) Priority: 02.03.2001 DK 200100341
(43) Date of publication of application: 26.11.2003
(73) Proprietor: A/S GEA Farmaceutisk Fabrik, 2650 Hvidovre (DK)
(72) Inventor: FELUMB, Niels, Christian, DK-2830 VIRUM (DK); HENRIKSEN, Kristian, Lund, DK-2860 Soborg (DK); PEDERSEN, Soren, Bols, DK-2650 Hvidovre (DK)
(74) Representative: Simonsen, Christian Rosendal
(86) International application number: PCT/DK2002/000134
(87) International publication number: WO 2002/069969

(56) References cited:
- WO-A-00/78288
- WO-A-01/58449
- WO-A-99/48499

## Description

### Field of the invention

The present invention is related to the manufacture of a pharmaceutical formulation for oral administration of paroxetine, which is a well-known drug having found widespread application in the treatment and prophylaxis of depression, anxiety, and several other disorders.

### Background of the invention

The generic name paroxetine covers the compound (-)-trans-4-(4'-flourophenyl)-3-(3',4'-methylene-dioxyphenoxymethyl)-piperidine which is a liquid base most conveniently handled in the form of an acid addition salt.

According to EP 0 223 403 B1, the hydrochloride of a basic compound is in general the preferred salt for therapeutical use because of its physiological acceptability.

Paroxetine hydrochloride exists in amorphous as well as crystalline forms. Several crystalline forms have been reported. Thus, WO 96/94595 describes four new forms. Furthermore, the hydrochloride forms quite stable solvates comprising organic solvents as well as at least one hydrate.

According to the above EP 0 223 403 B1, paroxetine hydrochloride hemihydrate is a relatively stable compound, from which the bound water, however, may be removed to give the anhydrous form when subjected to extreme dessication conditions. Said patent specification also discloses that paroxetine hydrochloride anhydrate when compressed is partly converted into the hemihydrate even in a relatively dry environment.

WO 95/16448 discloses that a pink discolouration had been experienced as a problem when aqueous granulation processes were used in connection with the tablet formulation of the hydrochloride hemihydrate. It also discloses that the hemihydrate may be formulated into tablets by using a process in which water is absent, such as by direct compression or by dry granulation, and that the tablets thus produced are less likely to develop a pink hue.

According to WO 99/58113 claiming priority from 13 May 1998, all paroxetine hydrochloride sold before that date has been in the form of tablets containing the hemihydrate, but it is possible to formulate the hydrochloride anhydrate into tablets without conversion into the hemihydrate provided that extremely dry conditions are used in a tableting process in which a completely dry granulation is used or in which the tablets are pressed directly from the powdery dry constituents, i.e. that essentially anhydrous excipients must be used.

In said WO 99/58113, the partial conversion of the hydrochloride anhydrate into the hydrochloride hemihydrate during the tableting process is described as creating difficulties in establishing and maintaining a reference standard for regulatory and quality control purposes.

However, formulation processes avoiding hemihydrate formation by using dry granulation or dry direct tablet pressing have certain drawbacks.

Thus, there is a risk of segregation of the mixture of the active paroxetine salt and the various adjuvants during the conveyance of the mixture from the blending device to the tablet matrix. This involves a risk that the tablets produced have a nonuniform content of active drug and/or that non-desired variations occur as to mechanical properties or solubility and release of the active component.

Furthermore, the granulation and pressing operations performed as "dry" processes involve application of a higher pressure than necessary when a wet granulation process is used, which higher pressure increases the risk for the paroxetine hydrochloride anhydrate being converted into another crystalline form or partially into hemihydrate thereby creating an uncertainty as to the actual composition of the final tablet.

In contrast to tablet manufacturing using wet granulation in which fine particles and dust are bound into the granules, the dry processes are dusting, and due to the etching character of paroxetine hydrochloride this necessitates extensive provisions to avoid respiratory health risks to the staff.

### Summary of the invention

The present invention is based on the recognition that it is possible to produce stable tablets containing crystalline paroxetine hydrochloride anhydrate by an alternative process which does not exhibit the drawbacks of the above tablet manufacturing processes using dry granulation or direct powder pressing.

Thus, it has turned out that tablets containing crystalline paroxetine hydrochloride anhydrate can be produced using a wet granulation method without conversion of the anhydrate into hemihydrate provided that a very fast drying of the granules is applied. Such fast drying is achieved by performing the drying in a process in which the material to be dried is fluidized in the drying air.

The process of the invention is characterized in the following steps:
- subjecting crystalline paroxetine hydrochloride anhydrate together with adjuvants comprising filler, disintegrant, binder, and water to a high-shear mixing operation,
- continuing the mixing to granulate the resulting mixture,
- fluidizing the resulting granulate in a flow of heated drying air to dry the granulate,
- continuing this drying until the water activity of the granulate has been reduced to 0.10-0.25 aw, when measured as described herein,
- optionally adding one or more further adjuvants,
- mixing a glidant into the granulate and,
- compressing the resulting mixture into tablets each having a pre-determined content of paroxetine hydrochloride anhydrate.

The term "glidant" is used herein in a broad sense also comprising adjuvants sometimes termed lubricants and agents improving the free flowing capability of the granulate.

By a preferred embodiment of the process, at least a part of the binder and at least a part of the water is added to a mixture of paroxetine hydrochloride anhydrate, filler, and disintegrant as an aqueous binder solution while said mixture is subjected to high-shear mixing.

Alternatively, a binder may as a dry material be included in the mixture of the paroxetine hydrochloride anhydrate, filler, and disintegrant, and the water added slowly to this mixture during mixing in a high-shear blender. However, a more efficient and faster dispersion of the binder on all particles forming the mixture is obtained when the binder is supplied dissolved in the aqueous granulation liquid.

The drying of the granulate while fluidized in the drying air may be performed using a conventional fluid bed dryer. As mentioned, the granulate is dried to a water activity between 0.10 and 0.25 aw. This means that the drying is more extensive than what is customary in connection with wet granulation as pre-treatment of materials to be compressed into tablets.

The water activity indicated here and in the attached claims is the one which is determined by using a device available from Novasina using the following procedure: Approximately 7 g granulate is placed in a chamber having a volume of approximately 20 ml. The chamber is sealed air-tight and kept at ambient temperature (20-25° C) for 30 min. The relative humidity of the air in the chamber is then recorded. The water activity of the granulate, expressed in the unit aw, is 1/100 of the relative humidity recorded for the air.

Preferably, the drying is continued until a water activity between 0.15 and 0.22 aw.

Even if the material is thus more dry than usual in tablet manufacture using wet granulation, the compression into tablets may be performed using less pressure than necessary in dry granulation or direct granulation processes. This is probably due to the fact that the binder is much better distributed than in said two processes.

The process of the invention may be performed using adjuvants and excipients of the type conventional when manufacturing tablets using a wet granulation pre-treatment.

A suitable filler may thus comprise one or more of the following substances: microcrystalline cellulose, mannitol, calcium phosphates, lactose, starch, sorbitol, and suchrose. In view of the teaching of WO 99/58113, cited above, that microcrystalline cellulose shall preferably be avoided in paroxetine hydrochloride anhydrate tablets, it is surprising that in the present process micro-crystlaline cellulose acts as a perfect adjuvant.

A suitable disintegrant may comprise one or more of the following substances: sodium starch glycolate, starch, gelatinated starch, crosprovidone, and micro crystalline cellulose.

A suitable binder comprises one or more of the following substances: polyvinyl pyrrolidone, gelatine, starch, methyl cellulose, hydroxypropylcellulose and copovidone.

A suitable glidant comprises one or more of the following substances: anhydrous colloidal silica, sodium stearyl fumarate, magnesium stearate, talc powder, and polyethylene glycol.

Very satisfactory results have been obtained using an embodiment wherein paroxetine hydrochloride anhydrate, mannitol, microcrystalline cellulose and sodium starch glycolate are subjected to high-shear mixing and simultaneously an aqueous solution of copovidone (Kolidon VA64) is added slowly and the mixing continued to obtain the desired granulation.

In this embodiment, the aqueous solution and, if necessary, further water are added in such an amount that a moisture content in the granulated mixture of 10-30% by weight is achieved before the drying is initiated. When other excipients are used, a moisture content outside these limits may be suitable.

An important feature is that this moisture is removed by a fast drying to avoid conversion of the paroxetine hydrochloride anhydrate into the hemihydrate.

The fluid bed drying may be performed as a continuos process or, preferably, batch-wise.

The drying periode shall preferably not exceed 3 h. It is more preferably less than 2 h and most preferably between 15 min. and 1 h.

Tablets produced by the present process have been stored for several months after which no detectable conversion of the crystalline paroxetine hydrochloride anhydrate had occurred. No hemihydrate was found and no conversion into other crystalline forms than the one of the starting material was detected.

Also the mechanical stability of the tablets was satisfactory. The crystalline hydrochloride anhydrate is reported as being hygroscopic. However, due to the fact that the paroxetine salt only constitutes a minor portion of the tablets, the hygroscopicity has no adverse effect on the stability and keeping qualities of the tablets when kept in normal air-tight containers or blister packings.

Preferably, the total weight of the tablets is between 100 and 750 mg and each contains from 10 to 60 mg paroxetine, calculated as the free base.

Analysis of the tablets indicated substantially the same content of paroxetine in each tablet, reflecting that no segregation had occurred during drying and compression operations.

As mentioned the tablets produced according to the invention show no tendency of discolouration during storing. However, since paroxetine has an unpleasant taste, it is preferred to subject the tablets to a film coating process. Such coating is not necessary to avoid discolouration or to secure sufficient stability of the tablets.

The binder in such a film coating may be methylhydroxypropyl cellulose, and water is used as solvent.

In contrast to what should be expected based on the teaching of the above cited prior art, also this contact between the crystalline paroxetine hydrochloride anhydrate and water takes place without conversion of the anhydrate into the hemihydrate.

### Detailed description of the invention

In the following, the process of the invention is further elucidated by means of an embodiment example.

### Example

22.22 kg crystalline paroxetine hydrochloride anhydrate, 80.0 kg microcrystalline cellulose PH101, 6.0 kg sodium starch glycolate, and 72.0 kg mannitol were introduced into a high-shear blender. After mixing of said four components in dry condition, an aqueous solution of 8.0 kg copovidone (Kolidon VA64) in 48.0 kg purified water was added slowly and the high-shear mixing continued to finish the granulation process.

The thus produced wet granulate was immediately transferred to a fluidized bed dryer and dried therein to a water activity of approximately 0.20 aw. The time period necessary to achieve this drying had been determined previously by guiding experiments. With the above stated composition of the wet granulate, the desired reduction of the water activity was obtained after drying in 1 h.

Subsequently, the dried granulate was sieved to remove lumps and afterwards transferred into a cone blender and therein mixed with 47.7 kg micro crystalline cellulose PH102, 0.48 kg anhydrous colloidal silica and 3.6 kg sodium stearyl fumarate.

The resulting dry mixture of granulate and said further adjuvants was compressed into tablets using a conventional rotary press having 16 pressing stations.

The pressing operation was carried out using a pressure lower than the one required in connection with direct powder pressing or pressing after dry granulation of similar materials. In spite thereof, the tablets were hard and had fine mechanical properties.

A total of 240 kg tablets, corresponding to 1 mio. pieces of tablets was produced, each comprising the same amount of crystalline paroxetine hydrochloride anhydrate, corresponding to 20 mg of the paroxetine base.

The tablets were film-coated using a coating liquid containing 1.382 kg methylhydroxypropyl cellulose (5), 0.806 kg micronized talc, 0.288 kg titanium dioxide and 26.324 kg purified water.

The tablets thus produced were subjected to several tests.

Stability studies of tablets packed in Al/PVC blister cards or polyethylene containers have been performed with satisfactory results. Also breakability studies have been performed.

Comparative dissolution tests have been made. The results show that more than 80% of the paroxetine is released from the film coated tablets within 10 min.

XRD studies have been performed on the finished product in order to confirm that no conversion of the crystalline paroxetine hydrochloride anhydrate to hemihydrate form takes place during manufacture and storage.

Also enantiomeric purity has been investigated. The results show that the content of (+)-paroxetine hydrochloride corresponds to less than 0.1% of the paroxetine hydrochloride content, meaning that the finished product is enantiomerically pure.

In bioavailability studies tablets produced as above and also similar tablets having a paroxetine content of 40 mg, were after coating compared with commercially available film coated tablets containing paroxetine hydrochloride hemihydrate and found bio-equivalent to these.

It was also observed that the tablets, whether coated or not, did not show any discolouration even after prolonged storage.

## Claims

1. A process for the manufacture of pharmaceutical tablets containing paroxetine hydrochloride anhydrate, **characterized in** subjecting crystalline paroxetine hydrochloride anhydrate together with adjuvants comprising filler, disintegrant, binder, and water to a high-shear mixing operation,
- continuing the mixing to granulate the resulting mixture,
- fluidizing the resulting granulate in a flow of heated drying air to dry the granulate,
- continuing this drying until the moisture content of the granulate has been reduced to such an extent that the water activity of the granulate corresponds to a value between 0.10 and 0.25 aw, if calculated as 1/100 of a relative humidity recordal obtained if using a device available from Novasina and a procedure in which approximately 7 g granulate is placed in a chamber having a volume of approximately 20 ml and the relative humidity of the air in the chamber is recorded after the chamber having been kept air-tight sealed at 20-25°C for 30 min,
- optionally adding one or more further adjuvants,
- mixing a glidant into this granulate, and
- compressing the resulting mixture into tablets each having a pre-determined content of paroxetine hydrochloride anhydrate.

2. A process according to claim 1, wherein at least a part of said binder and at least a part of said water is added as an aqueous binder solution to a mixture of paroxetine anhydrate chloride, filler and disintegrant while said mixture is subjected to high-shear mixing.

3. A process according to claim 1 or 2, wherein the granulate is dried to a water activity between 0.15 and 0.22 aw.

4. A process according to anyone of the preceding claims, wherein the filler comprises one or more of the following substances: microcrystalline cellulose, mannitol, calcium phosphates, lactose, starch, sorbitol, and succhrose.

5. A process according to anyone of the preceding claims, wherein the disintegrant comprises one or more of the following substances: sodium starch glycolate, starch, gelatinated starch, crospovidone, and micro crystalline cellulose.

6. A process according to anyone of the preceding claims, wherein the binder comprises one or more of the following substances: polyvinyl pyrrolidone, gelatine, starch, methyl cellulose, hydroxypropylcellulose and copovidone.

7. A process according to anyone of the preceding claims, wherein crystalline paroxetine hydrochloride anhydrate, mannitol, microcrystalline cellulose and sodium starch glycolate are subjected to high-shear mixing and simultaneously an aqueous solution of copovidone is added slowly to obtain the desired granulation.

8. A process according to claim 7, wherein said aqueous solution and, if necessary, further water, are added in such an amount that a moisture content in the granulated mixture of 10-30% by weight is obtained.

9. A process according to anyone of the above claims, wherein the tablets produced each has a weight between 100 and 750 mg and each contains from 10 to 60 mg paroxetine, calculated as the free base.

10. A process according to anyone of the preceding claims, wherein the tablets formed by the compressing are subjected to a coating operation using an aqueous coating liquid.

## Patentansprüche

1. Verfahren zur Herstellung von Arzneimitteln in Tablettenform, die Paroxetinhydrochloridanhydrat enthalten, **dadurch gekennzeichnet, daß** man Paroxetinhydrochloridanhydrat zusammen mit Adjuvants, das einen Füllstoff umfaßt, Zersetzungsmittel, Bindemittel und Wasser einem Mischverfahren mit hohen Scherkräften unterwirft,
- die Vermischung fortsetzt, um die erhaltene Mischung zu granulieren,
- das so erhaltene Granulat in einem Strom aus erhitzter Trocknungsluft fluidisiert, um das Granulat zu trocknen,
- die Trocknung fortsetzt, bis der Feuchtigkeitsgehalt des Granulats in so einem Umfang reduziert wurde, daß die Wasseraktivität des Granulats einem Wert zwischen 0,10 und 0,25 aw entspricht, wenn dieser als 1/100 der relativen Feuchtigkeit berechnet wird, die unter Verwendung einer Vorrichtung, welche kommerziell von Novasina erhältlich ist, und einem Verfahren, bei dem etwa 7 g Granulat in eine Kammer gelegt werden, die ein Volumen von etwa 20 ml aufweist und die relative Feuchtigkeit der Luft in der Kammer aufgezeichnet wird, nachdem die Kammer luftdicht verschlossen bei 20-25° C für 30 Minuten belassen wurde,
- gegebenenfalls einen oder mehrere Hilfsmittel zugibt,
- ein Gleitmittel mit diesem Granulat vermischt, und
- die so erhaltene Mischung zu Tabletten komprimiert, die je eine vorbestimmte Menge an Paroxetinhydrochloridanhydrat aufweisen.

2. Verfahren gemäß Anspruch 1, bei dem mindestens ein Teil des Bindemittels und mindestens ein Teil des Wassers als wässerige Bindemittellösung zu der Mischung aus Paroxetinhydroanhydratchlorid, Füllmittel und Zersetzungsmittel zugegeben wird, wobei die Mischung einem Mischverfahren mit hohen Scherkräften ausgesetzt wird.

3. Verfahren gemäß Anspruch 1 oder 2, bei dem das Granulat auf eine Wasseraktivität zwischen 0,15 und 0,22 aw getrocknet wird.

4. Verfahren gemäß irgendeinem der vorstehenden Ansprüche, bei dem das Füllmittel einen oder mehrere der folgenden Verbindungen enthält: mikrokristalline Zellulose, Mannitol, Kalziumphosphate, Laktose, Stärke, Sorbitol und Succhrose.

5. Verfahren gemäß irgendeinem der vorstehenden Ansprüche, bei dem das Zersetzungsmittel einen oder mehrere der folgenden Verbindungen umfaßt: Natriumstärkeglykolat, Stärke, gelatinierte Stärke, Crospovidon und Mikrokristalline Zellulose.

6. Verfahren gemäß irgendeinem der vorstehenden Ansprüche, bei dem das Bindemittel einen oder mehrere der folgenden Verbindungen umfaßt: Polyvinylpyrrolidon, Gelatine, Stärke, Methylzellulose, Hydroxypropylzellulose und Copovidon.

7. Verfahren gemäß irgendeinem der vorstehenden Ansprüche, bei dem kristallines Paroxetinhydrochloridanhydrat, Mannitol, Mikrokristalline Zellulose und Natriumstärkeglykolat einem Mischverfahren mit hohen Scherkräften unterworfen werden und gleichzeitig eine wässerige Lösung des Copovidon langsam zugegeben wird, um die gewünschte Granulierung zu erreichen.

8. Verfahren gemäß Anspruch 7, bei dem die wässerige Lösung und, soweit notwendig, ferner Wasser, in einer solchen Menge zugegeben werden, daß der Feuchtigkeitsgehalt der granulierten Mischung bei 10-30 Gew.-% erhalten wird.

9. Verfahren gemäß irgendeinem der vorstehenden Ansprüche, bei dem die Tabletten so hergestellt werden, daß jede ein Gewicht zwischen 100 und 750 mg aufweist und jede von 10 bis 60 mg Paroxetin enthält, das als freie Base berechnet wird.

10. Verfahren gemäß irgendeinem der vorstehenden Ansprüche, bei dem die Tabletten, die durch Komprimierung erhalten wurden, einem Beschichtungsverfahren unterworfen werden, bei dem eine wässerige Beschichtungsflüssigkeit verwendet wird.

## Revendications

1. Un procédé de fabrication de comprimés pharmaceutiques contenant du chlorhydrate de paroxétine anhydre, **caractérisé en ce que**
- on soumet du chlorhydrate de paroxétine anhydre cristallin en même temps que des adjuvants comprenant des charges, agents de désintégration, liants et eau à une opération de malaxage sous haut cisaillement,
- on poursuit le malaxage pour granuler le mélange résultant,
- on fluidise les granulés résultants dans un courant d'air de séchage chauffé pour sécher le granulé,
- on poursuit ce séchage jusqu'à ce que la teneur en humidité des granulés soit abaissée à un degré tel que l'activité en eau des granulés corresponde à une valeur comprise entre 0,10 et 0,25 ae, calculée comme étant le 1/100 de l'enregistrement de l'humidité relative obtenue si on utilisait un dispositif fourni par la Société Novasima et un procédé dans lequel on dispose environ 7 g de granulé dans une chambre ayant un volume d'environ 20 ml et que l'on enregistre l'humidité relative de l'air dans la chambre après que la chambre ait été maintenue hermétiquement obturée vis-à-vis de l'air à 20-25°C pendant 30 min.,
- on ajoute éventuellement un ou plusieurs adjuvants supplémentaires,
- on incorpore par malaxage un agent de glissement dans les granulés, et
- on compresse le mélange résultant en comprimés ayant chacune une teneur prédéterminée en chlorhydrate de paroxétine anhydre.

2. Un procédé selon la revendication 1, dans lequel on ajoute au moins une partie dudit liant et au moins une partie de ladite eau sous forme d'une solution aqueuse de liant à un mélange de chlorhydrate de paroxétine anhydre, de charge et de désintégrant tout en soumettant ledit mélange à un malaxage sous haut cisaillement.

3. Un procédé selon la revendication 1 ou 2, dans lequel on sèche le granulé jusqu'à obtention d'une activité de l'eau comprise entre 0,15 et 0,22 ae.

4. Un procédé selon l'une quelconque des revendications précédentes, dans lequel la charge comprend un ou plusieurs des produits suivants: cellulose microcristalline, mannitol, phosphates de calcium, lactose, amidon, sorbitol et saccharose.

5. Un procédé selon l'une quelconque des revendications précédentes, dans lequel l'agent désintégrant comprend un ou plusieurs des produits suivants: glycolate d'amidon et de sodium, amidon, amidon gélatinisé, crospovidone et cellulose microcristalline.

6. Un procédé selon l'une quelconque des revendications précédentes dans lequel le liant comprend un ou plusieurs des produits suivants: polyvinyl pyrrolidone, gélatine, amidon, méthyl cellulose, hydroxypropylcellulose et copovidone.

7. Un procédé selon l'une quelconque des revendications précédentes dans lequel on soumet le chlorhydrate de paroxétine anhydre cristallin, le mannitol, la cellulose microcristalline et le glycolate d'amidon et de sodium à un malaxage sous haut cisaillement et simultanément on ajoute lentement une solution aqueuse de copovidone pour obtenir la granulation désirée.

8. Un procédé selon la revendication 7, dans lequel on ajoute ladite solution aqueuse, et, si nécessaire, de l'eau supplémentaire en quantité telle qu'on obtienne une teneur en humidité dans le mélange granulé de 10 à 30 % en poids.

9. Un procédé selon l'une quelconque des revendications ci-dessus, dans lequel les comprimés obtenus ont chacun un poids compris entre 100 et 750 mg et chacun contient de 10 à 60 mg de paroxétine, exprimé en base libre.

10. Un procédé selon l'une quelconque des revendications précédentes, dans lequel on soumet les comprimés obtenus par compression à une opération d'enrobage en utilisant un liquide d'enrobage aqueux.
